# EUROPEAN PATENT APPLICATION

(11) **EP 3 141 281 A1**
(43) Date of publication of application: **15.03.2017**
(21) Application number: 16153355.9
(22) Date of filing: 29.01.2016
(51) Int. Cl.: A61N 5/06, A61B 17/22, A61B 17/50, A61B 17/3209, A61B 17/32

(54) **SKIN TREATMENT DEVICE**

(30) Priority: 09.09.2015 TW 104129843
(71) Applicant: Cal-Comp Electronics & Communications Company Limited, New Taipei City 22201 (TW); Kinpo Electronics, Inc., New Taipei City 22201 (TW)
(72) Inventor: KU, TE-KAI, 22201 SHENKENG DIST., NEW TAIPEI CITY (TW)
(74) Representative: 2K Patentanwälte Blasberg Kewitz & Reichel

(57) **Abstract**

A skin treatment device (10) includes: a shell body (1); a pressing bar (2), formed with a first end (21) mounted in the shell body (1) and a second end (22) protruded from one distal end of the shell body (1) and formed with a squeezing part (23) having a through hole (231); a bactericidal lamp (4), installed in the shell body (1) and including a bactericidal light source (42) capable of generating plural bactericidal light beams (41) passing the through hole (231); and a cover member (5), disposed at one distal end of the shell body (1), served to cover the second end (22) and including a light condensing lens (51) disposed corresponding to the through hole (231) and the bactericidal lamp (4). Accordingly, when the bacteria existed on the skin or in skin pores are exposed to the bactericidal light beams (41), the bacteria can be naturally deceased.

## Description

### BACKGROUND OF THE INVENTION

### Field of the Invention

The present invention relates to a skin treatment device, especially to a skin treatment device capable of removing and eliminating acnes or pimples on the skin.

### Description of Related Art

Acnes or pimples are often formed in a protruding status on the skin or cause an inflamed phenomenon, so the skin may not be as nice and smooth as desired. In order to removing and eliminating acnes and pimples, he/she could usually use hands to squeeze or apply some medicines, but using hands for squeezing would often lead to situations such as not sufficiently squeezing or poorly cleaning, and applying medicines would lead to situations such as slow effect or prolonging the treatment period. As such, there is an ultrasonic skin treatment device available in the market for replacing the conventional methods such as hand squeezing.

The conventional ultrasonic skin treatment device mainly includes a pressing bar and an oscillator, the oscillator is installed on the pressing bar and capable of generating oscillations with a frequency of one million or three millions per second, when the pressing bar is used for pressing on the area to be treated, the oscillator is able to drive the pressing bar to rapidly generate micro vibrations, thereby assisting the pressing bar to sufficiently remove and eliminate the acnes or pimples. Accordingly, the ultrasonic skin treatment device is provided with advantages of sufficiently squeezing, proper cleaning and rapidly achieving a curing effect.

However, the above-mentioned ultrasonic skin treatment device has following disadvantages: because the pressing bar is used for pressing and squeezing out acnes or pimples, wounds could be formed when the acnes or pimple are squeezed out from the skin, if the pressing bar itself or the operating environment contains bacteria, the user may be infected by the bacteria because of the wounds, and the acnes or pimples may be worsened, thus the performance of the ultrasonic skin treatment device is greatly affected.

Accordingly, the applicant of the present invention has devoted himself for improving the mentioned disadvantages.

### SUMMARY OF THE INVENTION

The present invention is to provide a skin treatment device, in which a bactericidal lamp is installed, the bactericidal lamp is able to generate bactericidal light beams, so when the bacteria existed on the skin or in skin pores is exposed to the bactericidal light beams, the bacteria can be naturally deceased, thereby enabling the skin treatment device to be provided with effects of effectively sterilizing, cleaning and prettifying skin.

Accordingly, the present invention provides a skin treatment device, which includes: a shell body; a pressing bar, formed with a first end and a second end opposite to the first end, wherein the first end is mounted in the shell body, the second end is protruded from one distal end of the shell body, the second end is formed with a squeezing part, and the squeezing part is formed with a through hole; and a bactericidal lamp, installed in the shell body and including a bactericidal light source, wherein the bactericidal light source is able to generate a plurality of bactericidal light beams capable of passing the through hole.

Accordingly, the present invention provides a skin treatment device, which includes: a shell body; a pressing bar, formed with a first end and a second end opposite to the first end, wherein the first end is mounted in the shell body, the second end is protruded from one distal end of the shell body, the second end is formed with a squeezing part, and the squeezing part is formed with a through hole; a bactericidal lamp, installed in the shell body and including a bactericidal light source, wherein the bactericidal light source is able to generate a plurality of bactericidal light beams capable of passing the through hole; and a cover member, disposed at one distal end of the shell body, served to cover the second end and including a light condensing lens, wherein the light condensing lens is disposed corresponding to the through hole and the bactericidal lamp.

Based on what has been disclosed above, the squeezing part is formed with a circular ring extended from one distal end of the second end, the through hole is formed at the interior of the circular ring, one distal end of the circular ring is able to be used for cleaning and removing dead cortex in a small area, and the through hole is able to be used for cleaning and eliminating acnes in a large area.

With the first end being served as a pivot for the ultrasonic oscillator, the second end is able to generate an amplifying effect with high-frequency patting, and the squeezing part is enabled to massage deeper skin, thus the acnes or pimples can be more easily squeezed out or oscillated out from the skin.

When the squeezing part and the through hole are utilized for squeezing acnes or pimples, because the bactericidal lamp generates the bactericidal light beams capable of passing the through hole, if the bactericidal light beams are blue, the bacteria on the skin surface or inside skin pores would naturally decease, if the bactericidal light beams are red, functions of increasing the growth of collagen, enhancing wound healing, firming skin, anti-inflammation and eliminating pimples are provided, thereby allowing the skin treatment device to be provided with effects of effectively sterilizing, cleaning and prettifying skin.

The light condensing lens of the cover member is disposed corresponding to the through hole and able to be served to modify the light distribution for enabling the energy of the bactericidal light beams passing the light condensing lens to be more evenly distributed, so the bactericidal light beams guided by the light condensing lens is provided with the optimal illumination energy and evenness, so when the bactericidal light beams guided by the light condensing lens are projected to the skin, the inflamed skin can be effectively soothed and cured.

### BRIEF DESCRIPTION OF DRAWING

FIG. 1 is a perspective exploded view showing a skin treatment device according to the present invention;
FIG. 2 is another perspective exploded view showing the skin treatment device according to the present invention;
FIG. 3 is a cross sectional view showing the skin treatment device according to the present invention;
FIG. 4A is a cross sectional view showing a light guiding lens according to a first embodiment of the present invention;
FIG. 4B is a cross sectional view showing the light guiding lens according to a second embodiment of the present invention;
FIG. 5A is a cross sectional view showing a light condensing lens according to a first embodiment of the present invention;
FIG. 5B is a cross sectional view showing the light condensing lens according to a second embodiment of the present invention;
FIG. 5C is a cross sectional view showing the light condensing lens according to a third embodiment of the present invention;
FIG. 6 is a schematic view showing an operating status of the skin treatment device according to the present invention;
FIG. 7 is a schematic view showing another operating status of the skin treatment device according to the present invention;
FIG. 8 is a schematic view showing one another operating status of the skin treatment device according to the present invention; and
FIG. 9 is a schematic view showing still one another operating status of the skin treatment device according to the present invention.

### DETAILED DESCRIPTION OF THE INVENTION

Preferred embodiments of the present invention will be described with reference to the drawings.

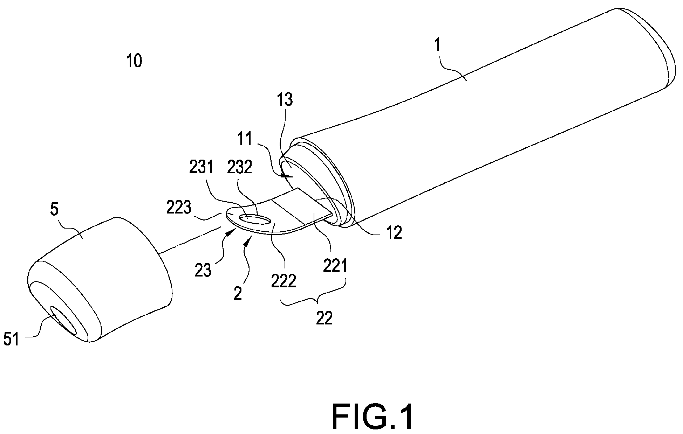

Please refer from FIG.1 to FIG. 9, the present invention provides a skin treatment device. The skin treatment device (10) mainly includes a shell body (1), a pressing bar (2), a bactericidal lamp (4) and a cover member (5).

As shown from FIG. 1 to FIG. 3, according to this embodiment, the shell body (1) is formed as an elongated shell member, what shall be addressed is that the scope of the present invention is not limited to the above-mentioned arrangement, the shell body (1) can also be formed as a shell member having any geometrical shape. In addition, one distal end of the shell body (1) is formed with an end surface (11), and the end surface (11) is formed with a penetrated hole (12) and a light pervious part (13).

As shown from FIG. 1 to FIG. 3, the pressing bar (2) is received in the penetrated hole (12) and formed with a first end (21) and a second end (22) opposite to the first end (21), the first end (21) is mounted in the shell body (1), the second end (22) is protruded from the distal end of the shell body (1), the second end (22) is formed with a squeezing part (23), and the squeezing part (23) is formed with a through hole (231).

Details are provided as follows. The second end (22) is formed with a straight segment (221) and an inclined segment (222) extended from one distal end of the straight segment (221), an obtuse angle (θ1) is formed between the straight segment (221) and the inclined segment (222), the obtuse angle (θ1) is between 140° to 165°, and the squeezing part (23) is formed on the inclined segment (222).

Moreover, the squeezing part (23) is formed with a circular ring (223) extended from one distal end of the inclined segment (222), the through hole (231) is formed at the interior of the circular ring (223), the through hole (231) is formed with an inner wall (232), one side of the inner wall (232) is formed with a bottom edge (233) close to the shell body (1), and another side thereof is formed with a top edge (234) away from the shell body (1).

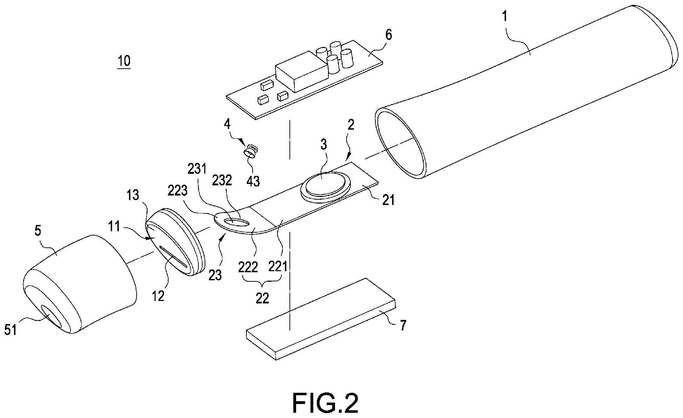

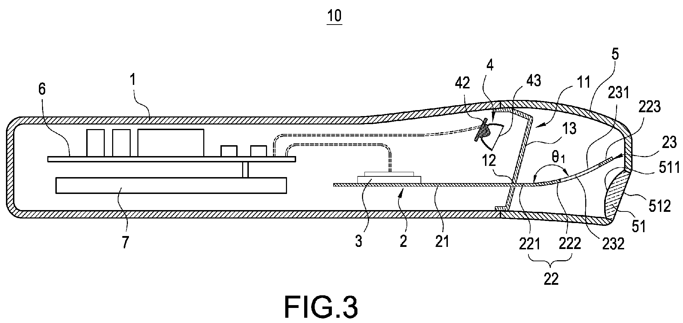

As shown in FIG. 2 and FIG. 3, the skin treatment device (10) further includes an ultrasonic oscillator (3). The ultrasonic oscillator (3) is installed at the first end (21) and capable of generating oscillations with a frequency of one million or three millions per second, so when the squeezing part (23) is used for performing a squeezing operation, the ultrasonic oscillator (3) is able to drive the squeezing part (23) to rapidly generate micro vibrations.

As shown in FIG. 2 and FIG. 3, the bactericidal lamp (4) is installed in the shell body (1) and includes a bactericidal light source (42). The bactericidal light source (42) is able to generate a plurality of bactericidal light beams (41) capable of passing the through hole (231). A light axis (L) is defined by the bactericidal light source (42), and an acute angle (θ2) is formed between the light axis (L) and the squeezing part (23), and the acute angle (θ2) is between 25°to 60°.

Wherein, the bactericidal light source (42) includes a blue-light LED (light emitting diode) or a red-light LED, the bactericidal light beams (41) can be blue lights with the wavelength of 415nm generated by the blue-light LED, when the bacteria existed on the skin or in skin pores is exposed to the blue lights with the wavelength of 415nm, and the light sensitive substance "porphyrin" contained in the acne bacteria is stimulated to absorb the blue lights thereby causing the acne bacteria to naturally decease; the bactericidal light beams (41) can also be red lights with the wavelength of 660nm+/-30nm generated by the red-light LED, and the red lights with the wavelength of 660nm+/-30nm has functions of increasing the growth of collagen, enhancing wound healing, firming skin, anti-inflammation and eliminating pimples according to the dermatology.

Details are provided as follows. The bactericidal lamp (4) further includes a light guiding lens (43). The light guiding lens (43) is disposed on top of the bactericidal light source (42) and used for guiding a part of the bactericidal light beams (41) to shift from the bottom edge (233) towards the top edge (234), in other words a part of the bactericidal light beams (41) projected to the bottom edge (233) are altered to be projected to the top edge (234).

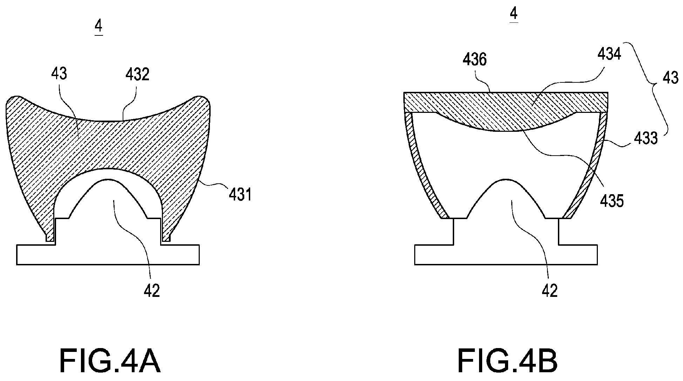

In addition, as shown in FIG. 4A, which discloses a first embodiment of the light guiding lens (43) provided by the present invention; the outer peripheral wall of the light guiding lens (43) is formed with a bowl-shaped reflection wall (431) and the top surface thereof is formed with a concave arc-shaped light pervious surface (432). The bowl-shaped reflection wall (431) is used for reflecting and guiding light for being projected to the concave arc-shaped light pervious surface (432), so a light gathering effect is provided when the light passes the concave arc-shaped light pervious surface (432).

In addition, as shown in FIG. 4B, which discloses a second embodiment of the light guiding lens (43) provided by the present invention; the light guiding lens (43) includes a bowl-shaped reflection sheet (433) and a light pervious sheet (434). The bowl-shaped reflection sheet (433) is disposed between the bactericidal light source (42) and the light pervious sheet (434), the light pervious sheet (434) is formed with a convex arc-shaped surface (435) and a planar surface (436) opposite to the convex arc-shaped surface (435). The convex arc-shaped surface (435) is disposed corresponding to the bactericidal light source (42), the bowl-shaped reflection sheet (433) is used for reflecting and guiding light for being projected to the light pervious sheet (434), so a light gathering effect is provided when the light passes the convex arc-shaped surface (435).

As shown from FIG. 1 to FIG. 3, the cover member (5) is disposed at one distal end of the shell body (1), served to cover the second end (22) and includes a light condensing lens (51). The light condensing lens (51) is disposed corresponding to the through hole (231) and the bactericidal lamp (4).

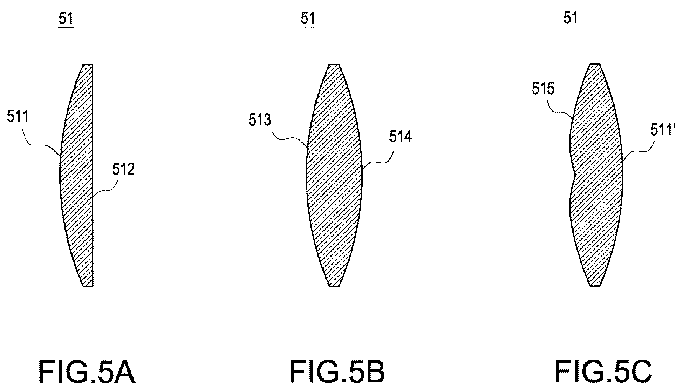

Details are provided as follows. As shown in FIG. 5A, which discloses a first embodiment of the light condensing lens (51) provided by the present invention; the light condensing lens (51) is formed with a convex curved surface (511) and a light diffusing surface (512) opposite to the convex curved surface (511). The convex curved surface (511) is disposed corresponding to the through hole (231), so a light gathering effect is provided when the light passes the convex curved surface (511).

In addition, as shown in FIG. 5B, which discloses a second embodiment of the light condensing lens (51) provided by the present invention; the light condensing lens (51) includes a first convex curved surface (513) and a second convex curved surface (514) opposite to the first convex curved surface (513). The first convex curved surface (513) is disposed corresponding to the through hole (231), so a light gathering effect is provided when the light passes the first convex curved surface (513) and the second convex curved surface (514).

Moreover, as shown in FIG. 5C, which discloses a third embodiment of the light condensing lens (51) provided by the present invention; the light condensing lens (51) includes a convex curved surface (511') and an aspheric surface (515) opposite to the convex cured surface (511'). The aspheric surface (515) is disposed corresponding to the through hole (231), so a light gathering effect is provided when the light passes the aspheric surface (515) and the convex curved surface (511').

As shown in FIG. 2 and FIG. 3, the skin treatment device (10) provided by the present invention further includes a circuit board (6) and a battery (7). The circuit board (6) and the battery (7) are respectively accommodated in the shell body (1). The circuit board (6) is electrically connected to the battery (7), and the circuit board (6) is electrically connected to the ultrasonic oscillator (3) and the bactericidal light source (42).

As shown in FIG. 3, one assembly of the skin treatment device (10) provided by the present invention is illustrated as follows: the pressing bar (2) is formed with the first end (21) and the second end (22) opposite to the first end (21), the first end (21) is mounted in the shell body (1), the second end (22) is protruded from the distal end of the shell body (1), the second end (22) is formed with the squeezing part (23), and the squeezing part (23) is formed with the through hole (231); the bactericidal lamp (4) is installed in the shell body (1) and includes the bactericidal light source (42), the bactericidal light source (42) is able to generate the bactericidal light beams (41) capable of passing the through hole (231).

As shown in FIG. 3, another assembly of the skin treatment device (10) provided by the present invention is illustrated as follows: the pressing bar (2) is formed with the first end (21) and the second end (22) opposite to the first end (21), the first end (21) is mounted in the shell body (1), the second end (22) is protruded from the distal end of the shell body (1), the second end (22) is formed with the squeezing part (23), and the squeezing part (23) is formed with the through hole (231); the bactericidal lamp (4) is installed in the shell body (1) and includes the bactericidal light source (42), the bactericidal light source (42) is able to generate the bactericidal light beams (41) capable of passing the through hole (231); the cover member (5) is disposed at the distal end of the shell body (1) and served to cover the second end (22), the cover member (5) includes the light condensing lens (51), and the light condensing lens (51) is disposed corresponding to the through hole (231) and the bactericidal lamp (4).

As shown in FIG. 3, FIG. 6 and FIG. 7, a first operating status of the skin treatment device (10) provided by the present invention is illustrated as follows: when the squeezing part (23) and the through hole (231) are utilized for squeezing acnes or pimples, the skin treatment device (10) further includes the ultrasonic oscillator (3), the ultrasonic oscillator (3) is installed at the first end (21), because the first end (21) is served as a pivot for the ultrasonic oscillator (3), the second end (22) is able to generate an amplifying effect with high-frequency patting, and the squeezing part (23) is enabled to massage deeper skin, thus the acnes or pimples can be more easily squeezed out or oscillated out from the skin.

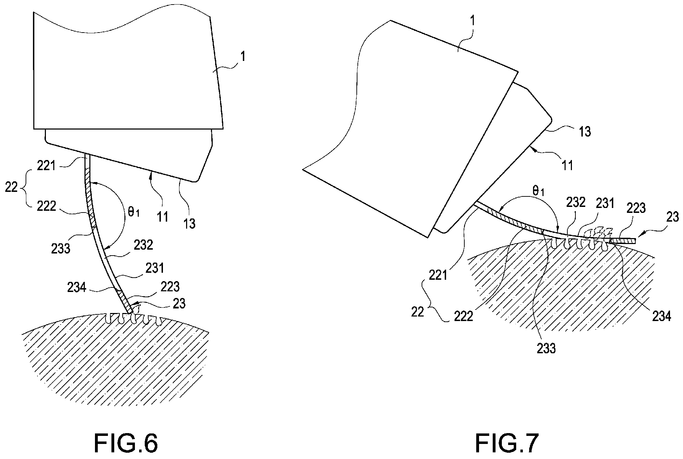

In addition, the squeezing part (23) includes the circular ring (223) extended from the distal end of the inclined segment (222), and the through hole (231) is formed at the interior of the circular ring (223). As shown in FIG. 6, the distal end of the circular ring (223) is able to be used for cleaning and removing dead cortex in a small area; as shown in FIG. 7, the through hole (231) is able to be used for cleaning and eliminating acnes in a large area.

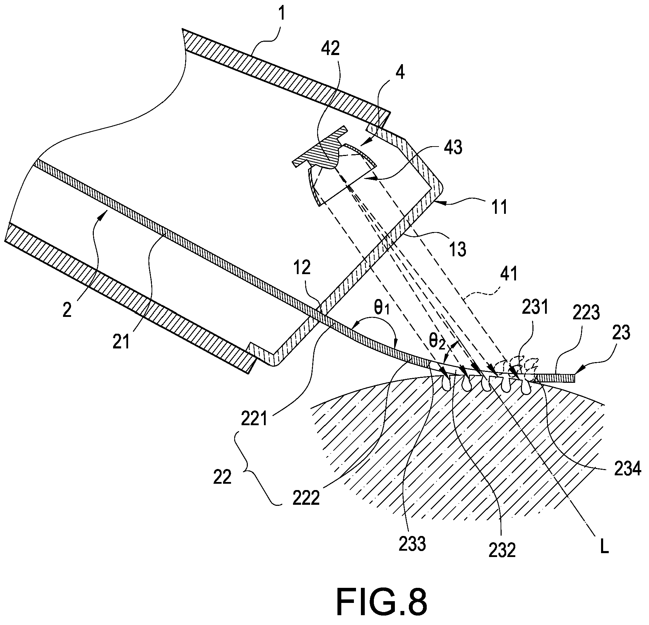

As shown in FIG. 8, a second operating status of the skin treatment device (10) provided by the present invention is illustrated as follows: when the squeezing part (23) and the through hole (231) are utilized for squeezing acnes or pimples, because the bactericidal light source (42) generates the bactericidal light beams (41) capable of passing the through hole (231), if the bactericidal light beams (41) are blue, the bacteria on the skin surface or inside skin pores would naturally decease, if the bactericidal light beams (41) are red, functions of increasing the growth of collagen, enhancing wound healing, firming skin, anti-inflammation and eliminating pimples are provided, thereby allowing the skin treatment device (10) to be provided with effects of effectively sterilizing, cleaning and prettifying skin.

In addition, the bactericidal lamp (4) further includes the light guiding lens (43), the light guiding lens (43) is disposed on top of the bactericidal light source (42), so the bactericidal light beams (41) can be gathered through the light guiding lens (43) for being evenly projected to an area defined by the through hole (231), thereby enabling the skin treatment device (10) to be provided with effects of cleaning skin, sterilizing and prettifying skin.

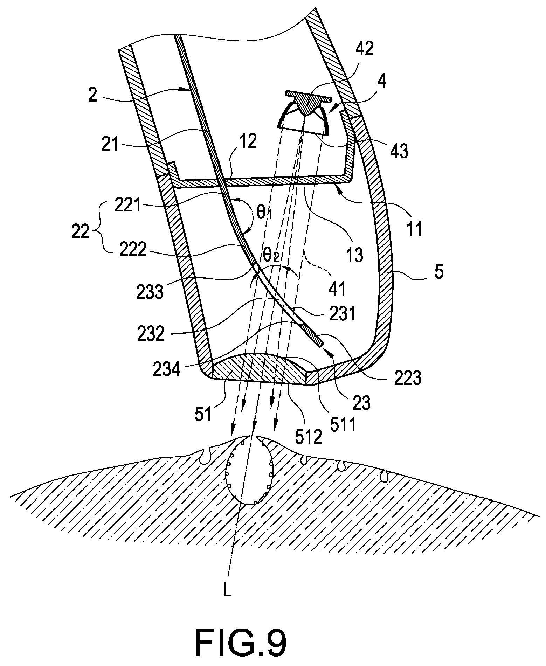

As shown in FIG. 9, a third operating status of the skin treatment device (10) provided by the present invention is illustrated as follows: the cover member (5) is assembled on the shell body (1), the light condensing lens (51) of the cover member (5) is disposed corresponding to the through hole (231), because the light guiding lens (43) is served to guide a part of the bactericidal light beams (41) to shift from the bottom edge (233) towards the top edge (234), an energy unevenly distributing phenomenon is generated when the bactericidal light beams (41) are shifted to the cover member, at this moment the light condensing lens (51) is served to modify the light distribution for enabling the energy of the bactericidal light beams (41) passing the light condensing lens (51) to be more evenly distributed, so the bactericidal light beams (41) guided by the light condensing lens (51) is provided with the optimal illumination energy and evenness, so when the bactericidal light beams (41) guided by the light condensing lens (51) are projected to the skin, the inflamed skin can be effectively soothed and cured.

## Claims

1. A skin treatment device, including:
a shell body (1);
a pressing bar (2), formed with a first end (21) and a second end (22) opposite to the first end (21), wherein the first end (21) is mounted in the shell body (1), the second end (22) is protruded from one distal end of the shell body (1), the second end (22) is formed with a squeezing part (23), and the squeezing part (23) is formed with a through hole (231); and
a bactericidal lamp (4), installed in the shell body (1) and including a bactericidal light source (42), wherein the bactericidal light source (42) is able to generate a plurality of bactericidal light beams (41) capable of passing the through hole (231).

2. The skin treatment device according to claim 1, further including an ultrasonic oscillator (3), a circuit board (6) and a battery (7), wherein the ultrasonic oscillator (3) is installed at the first end (21), the circuit board (6) and the battery (7) are respectively accommodated in the shell body (1), the circuit board (6) is electrically connected to the battery (7), and the circuit board (6) is electrically connected to the ultrasonic oscillator (3) and the bactericidal light source (42).

3. The skin treatment device according to claim 1 or 2, wherein a light axis (L) is defined by the bactericidal light source (42), an acute angle (θ2) is formed between the light axis (L) and the squeezing part (23), the acute angle is between 25° to 60°, and the bactericidal light source (42) includes a blue-light LED or a red-light LED.

4. The skin treatment device according to any of the claims 1 to 3, wherein the through hole (231) is formed with an inner wall (232), one side of the inner wall (232) is formed with a bottom edge (233) defined close to the shell body (1), and another side thereof is formed with a top edge (234) defined away from the shell body (1), the bactericidal lamp (4) further includes a light guiding lens (43), the light guiding lens (43) is disposed on top of the bactericidal light source (42) and used for guiding a part of the bactericidal light beams (41) to shift from the bottom edge (233) towards the top edge (234).

5. The skin treatment device according to claim 4, wherein the outer peripheral wall of the light guiding lens (43) is formed with a bowl-shaped reflection wall (431) and the top surface thereof is formed with a concave arc-shaped light pervious surface (432).

6. The skin treatment device according to claim 4 or 5, wherein the light guiding lens (43) includes a bowl-shaped reflection sheet (433) and a light pervious sheet (434), the bowl-shaped reflection sheet (433) is disposed between the bactericidal light source (42) and the light pervious sheet (434), the light pervious sheet (434) is formed with a convex arc-shaped surface (435) and a planar surface (436) opposite to the convex arc-shaped surface (435), and the convex arc-shaped surface (435) is disposed corresponding to the bactericidal light source (42).

7. The skin treatment device according to any of the claims 1 to 6, wherein one distal end of the shell body (1) is formed with an end surface (11), the end surface (11) is formed with a penetrated hole (12) and a light pervious part (13), the pressing bar (2) is received in the penetrated hole (12), and the bactericidal light source (42) is disposed corresponding to the light pervious part (13).

8. The skin treatment device according to any of the claims 1 to 7, wherein the second end (22) is formed with a straight segment (221) and an inclined segment (222) extended from one distal end of the straight segment (221), an obtuse angle (θ1) is formed between the straight segment (221) and the inclined segment (222), the obtuse angle (θ1) is between 140° to 165°, the squeezing part (23) is formed on the inclined segment (222), the squeezing part (23) includes a circular ring (223) extended from one distal end of the inclined segment (222), and the through hole (231) is formed at the interior of the circular ring (223)..

9. The skin treatment device according to any of the claims 1 to 8, further including a cover member (5) disposed at one distal end of the shell body (1), served to cover the second end (22) and including a light condensing lens (51), wherein the light condensing lens (51) is disposed corresponding to the through hole (231) and the bactericidal lamp (4).

10. The skin treatment device according to claim 9, wherein the light condensing lens (51) is formed with a convex curved surface (511) and a light diffusing surface (512) opposite to the convex curved surface (511), and the convex curved surface (511) is disposed corresponding to the through hole (231).

11. The skin treatment device according to claim 9 or 10, wherein the light condensing lens (51) includes a first convex curved surface (513) and a second convex curved surface (514) opposite to the first convex curved surface (513).

12. The skin treatment device according to any of the claims 9 to 11, wherein the light condensing lens (51) includes a convex curved surface (511') and an aspheric surface (515) opposite to the convex curved surface (511'), and the aspheric surface (515) is disposed corresponding to the through hole (231).
